Europäisches Patentamt

European Patent Office

Office européen des brevets

(11) Publication number: **0 220 803**
**A2**

## EUROPEAN PATENT APPLICATION

(21) Application number: **86306213.9**

(51) Int. Cl.⁴: **A61F 2/32**

(22) Date of filing: **12.08.86**

(30) Priority: **16.09.85 US 776303**

(43) Date of publication of application:
**06.05.87 Bulletin 87/19**

(84) Designated Contracting States:
**CH DE FR GB IT LI**

(71) Applicant: **DOW CORNING CORPORATION**

**Midland Michigan 48640(US)**

(72) Inventor: **Brooks, John Granville, Jr.**
**390 Bouldincrest**
**Collierville Tennessee(US)**
Inventor: **Kaufman, Michael Earl**
**6024 Tiffany Road**
**Bartlett Tennessee(US)**

(74) Representative: **Lewin, John Harvey et al**
**Elkington and Fife High Holborn House 52/54**
**High Holborn**
**London WC1V 6SH(GB)**

(54) **Multiple component hip femoral prosthesis.**

(57) Disclosed herein is a multiple component hip femoral prosthesis system having a stem component (11) for intramedullary implantation in a truncated femur, a neck component (12) connected to the stem component (11) by means of a stud (18) projecting from one of the components and mating with a recess (20) provided in the other for reciprocating movement therein, a rigid calcar collar (14) slideably positioned on the stud (18) and designed to overlie the cut end of the bone including at least a substantial portion of the calcar (14), a cushioning body (15) of elastomeric material such as silicone rubber interposed between the calcar collar (14) and a shoulder (19) on the neck component (12), and a ball-shaped femoral head (13) preferably detachably mounted on the upper end of the neck component (12) for articulation with the acetabulum or an acetabular prosthesis. The stud (18) is enabled to slide up and down in the mating recess (20) as stresses are applied and relieved. The cushioning body (15) absorbs shock and causes forces to be substantially evenly distributed over the calcar collar (14) which in turn distributes forces over a large bone area to prevent localized stresses and bone resorption and the stem (11) is effectively isolated from shock forces.

## MULTIPLE COMPONENT HIP FEMORAL PROSTHESIS

The present invention relates to surgically implantable hip prostheses and more particularly to improvements in femoral components of such devices.

Hip arthoplasty has now become commonplace to repair hip joints which have become damaged due to trauma or arthritic conditions, for example. In such surgery, the femoral head is removed and replaced by an artificial, generally ball-shaped, member mounted on a stem which is implanted in the proximal end of the truncated femur.

In most cases at present, acrylic cement is used to hold the prosthesis stem in place. Cement has been chosen as a means of distributing forces transmitted from prosthesis to bone to prevent localized stresses in the hope of avoiding bone resorption and subsequent loosening of the prosthesis. It has been found however, that frequently the cement mantle eventually fractures due to stresses.

Another approach which has been taken to prevent resorption and loosening is the provision of a porous surface on the prosthesis to allow bone or other tissue ingrowth into the surface of the prosthesis.

A third approach has been an attempt to provide cushioning material such as a silicone rubber coating on the prosthesis stem. This is shown, for example, by Kahn et. al., U.S. Patent No. 3,938,198 in which provision is also made for tissue ingrowth. There have been reports of abrasion of exposed cushioning materials by adjacent bone or tearing of the material perhaps by torsional or shear forces being applied.

Various other approaches to stress distribution and shock absorption have been proposed but the problem of implant loosening has not been completely solved.

A different kind of problem is faced if a patient develops a need for replacement of an acetabular cup with a different size inner diameter in that the femoral component must also be replaced, which in most prior art devices requires removal of the entire device. It is, therefore, desirable to provide for replacement of the ball-shaped member without disturbing the implanted stem. Interchangeable ball-shaped members also provide the advantage of reducing inventory needs for both manufacturer and user.

The present invention is aimed at alleviating the aforementioned disadvantages inherent in prior art femoral hip prostheses. In particular, it is designed to provide a shock absorbing feature to provide attenuation of peak stresses which tend to cause fatigue failure of the cement mantle, if cement is used, or bone resorption and loosening if other methods of stem fixation are included. Further, the invention is designed to provide for interchangeability of head components so that the same stem may be fitted with alternate sizes or the head replaced if damaged.

The prosthesis of this invention comprises at least two and preferably three or more components which are interchangeable. The first component is a more or less conventional stem portion designed to be implanted into a reamed intramedullary canal of a resected femur. The proximal end of the stem portion is designed to be positioned at or slightly below the level of the cut end of the femur. There is provided in the proximal end of the stem portion a recess designed to mate with a stud protruding from the bottom of a neck component, or alternatively the stud may protrude from the upper surface of the stem portion and mate with a recess in the bottom of the neck component. There is preferably interposed around the stud between the neck component and the stem a rigid calcar collar designed to overlie a substantial portion of the cut bone end including the calcar of the resected femur. Between the collar and a shoulder at the bottom of the neck component is a cushioning body of elastomeric material such as silicone rubber. If the calcar collar is not used, the cushioning body is interposed directly between the neck component and the proximal end of the stem component. The upper or proximal portion of the neck component is fitted with a ball-shaped femoral head designed to articulate with the acetabulum or an acetabular cup prosthesis. The ball-shaped femoral head is preferably fitted with a tapered recess designed to mate with a tapered top portion of the neck component to provide for easy interchangeability of femoral head members. Alternatively, the femoral head member can be made integrally with the neck component.

In use, the stem portion is implanted so that its proximal end lies at or slightly below the truncated end of the femur. The calcar collar contacts the cut end of the bone and the cushioning material positioned between the collar and the lower end of the neck component compresses under stress allowing pistoning of the stud of the neck component in the recess of the stem proximal end thereby isolating the stem from direct transmission of forces as is typical of state-of-the-art femoral prosthesis designs. The calcar collar in combination with the cushioning provides substantial distribution of stresses across the entire area of the collar to the cut end of the bone including the calcar. This limits the likelihood of bone resorption due to localized stresses on the bone and minimizes the risk of loosening of the prosthesis. The location of the cushioning material between two relatively rigid bodies also protects the cushioning material from abrasion by bone or the other prosthesis components and the overall design insures that the primary stresses on the cushioning material are compressive so that torsional or shearing stresses which might damage the relatively soft material are minimized.

The invention will become better understood by those skilled in the art from a consideration of the following detailed description when read in connection with the accompanying drawings wherein:

FIG. 1 is an elevational view of a femoral prosthesis in accordance with the present invention, and

FIG. 2 is a view similar to FIG. 1 but shown in partial cross-section to show details of the construction of the prosthesis of FIG. 1.

Referring now to the drawings, wherein the same reference characters represent the same parts in both the figures, there is shown a multiple component femoral prosthesis system comprising a stem 11 designed to be implanted into a reamed intramedullary canal of a truncated femur. A neck component 12 having a ball-shaped femoral head 13 for articulation with the patients' acetabulum or a prosthetic acetabular cup is mounted on the upper portion of the stem 11 as will be described below. A calcar collar 14 of rigid material and a cushioning body 15 of elastomeric material are interposed between the calcar collar 14 and an enlarged shoulder 19 at the lower end of the neck component 12. If desired, the calcar collar can be omitted and the shoulder 19 reduced to the approximate dimensions of the proximal end of the stem whereby the cushioning body is interposed directly between the neck component 12 and the stem. The stem portion 11, calcar collar 14, neck component 12 and femoral head 13 may be made of any high strength rigid materials suitable for use in permanently implanted orthopedic applications, such as cobalt-chrome or titanium alloys. The stem may be provided with a conventional porous coating for bone ingrowth or for better cement adhesion, if desired. The cushioning body 15 is desirably a medically acceptable silicone-containing polymeric material such as silicone rubber, or other viscoelastic polymers or copolymers.

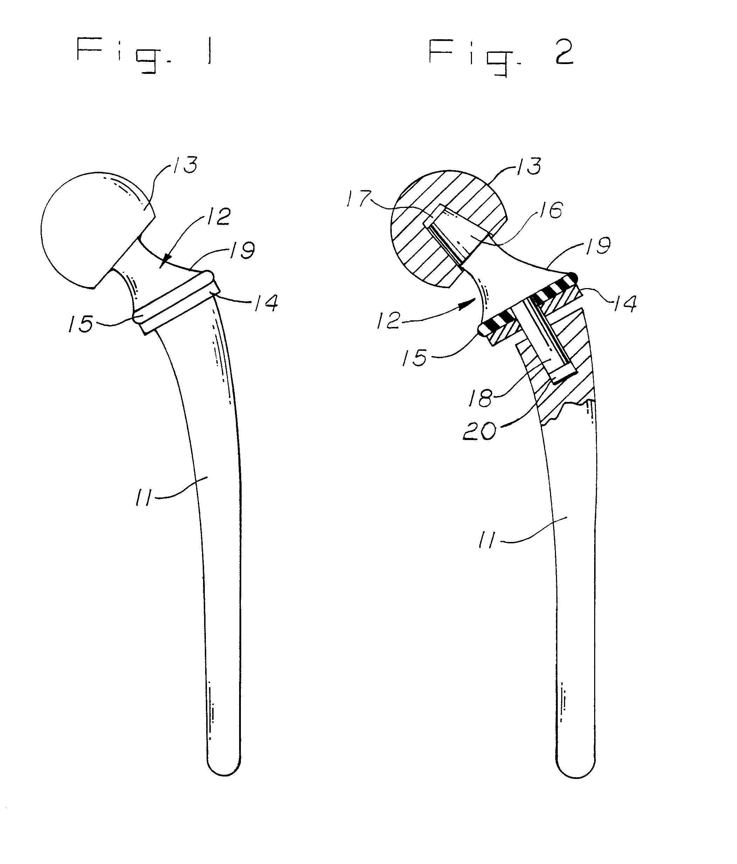

Referring now to FIG. 2, the prosthesis is preferably formed with a taper portion 16 fitted into a recess 17 in the femoral head 13 having a similar taper so that the femoral head may be easily removed and the neck portion fitted with a femoral head of a different size but having a similar tapered recess.

As may also be seen from FIG. 2, the neck component 12 is connected to the stem portion 11 by means of a stud 18 positioned in a recess 20 which is deeper than the length of stud normally extending below the calcar collar 14 to allow a pistoning movement of the stud in the recess. As can be seen further, the stud 18 extends through the cushioning body 15 and the calcar collar 14 and is also freely movable with respect to the calcar collar. The cushioning body 15 is confined between the collar and the enlarged shoulder 19 at the lower end of the neck component. As an alternative construction, the stud can be provided on the top of the stem component and the mating recess provided in the bottom of the neck component to provide similar function. The stud and/or recess can, if desired, be provided with a surface layer of cushioning material.

In use, the stem portion 11 is implanted in the reamed intramedullary canal of the resected femur of the patient in a manner such that its proximal end lies even with or slightly below the cut bone end so that the calcar collar 14 rests against the cut end of the bone. Forces applied to the femoral head by walking or other activity of the patient cause compression of the cushioning body 15 between the calcar collar and the surface presented by the shoulder 19 of the neck component resulting in pistoning movement of the stud 18 in its mating recess 20 as forces are applied and released. The cushioning body acts to dampen shocks or sudden forces and to distribute applied forces across the calcar collar. The calcar collar in turn transmits the dampened distributed forces substantially evenly across the calcar and other cancellous bone surrounding the implant tending to insure against uneven stresses and subsequent bone resorption. Similarly, if the calcar collar is omitted, the cushioning body confined between the neck component and the proximal end of the stem will still act to dampen shocks or sudden forces and reduce subsequent problems.

In the event that further surgical intervention on the hip becomes necessary, e.g. to correct problems in the acetabular area, a new femoral head may be installed by simple removal of the old one without disturbing the fixed implant stem as was necessary in prior art devices. Similarly, replacement of the cushioning body and calcar collar can be accomplished by removing the neck component from the stem.

Obviously, variations and modifications of the described preferred embodiment will occur to those skilled in the art from a reading of the above description. It is, therefore, to be understood that within the scope of the appended claims the invention may be practiced otherwise than as specifically described.

## Claims

1. A multiple component hip femoral prosthesis comprising:

a stem component for implantation in the intramedullary canal of a truncated femur,

a neck component,

said stem and neck components having a stud projecting from one component inserted in a mating recess in the other of said components for reciprocating movement therein,

a cushioning body of elastomeric material interposed between the stem and the neck components, and

a substantially ball-shaped femoral head positioned on the upper end of the neck component for articulation with the acetabulum or an acetabular cup prosthesis.

2. A multiple component hip femoral prosthesis as defined in Claim 1 wherein the cushioning body is silicone-containing polymeric material.

3. A multiple component hip femoral prosthesis as defined in either Claim 1 or Claim 2 wherein the top of the neck component is provided with a portion having an upwardly tapering cross-section which mates with a similarly tapered recess in the bottom of the femoral head, whereby a different femoral head may be substituted.

4. A multiple component hip femoral prosthesis as defined in Claim 1 wherein said neck component has a shoulder at its lower end, said prosthesis further includes a calcar collar of rigid material slideably positioned on said stud and shaped to contact a substantial portion of the cut end of the bone adjacent the stem, and said cushioning body is interposed between the calcar collar and the shoulder of the neck component.

5. A multiple component hip femoral prosthesis as defined in Claim 4 wherein the cushioning body is silicone-containing polymer material.

6. A multiple component hip femoral prosthesis as defined in either Claim 4 or Claim 5 wherein the top of the neck component is provided with a portion having an upwardly tapering cross-section which mates with a similarly tapered recess in the bottom of the femoral head, whereby a different femoral head may be substituted.

Fig. 1

Fig. 2